# FASCICULE DE BREVET EUROPEEN

(11) **EP 0 923 931 B1**
(45) Date de publication et mention de la délivrance du brevet: **12.07.2000**
(21) Numéro de dépôt: 98402941.3
(22) Date de dépôt: 25.11.1998
(51) Int. Cl.: A61K 7/48

(54) **Composition cosmétique solide et ses utilisations**
Feste kosmetische Zusammensetzung sowie ihre Verwendungen
Solid cosmetic composition and uses thereof

(30) Priorité: 19.12.1997 FR 9716174
(43) Date de publication de la demande: 23.06.1999
(73) Titulaire: L'OREAL, 75008 Paris (FR)
(72) Inventeur: Roulier, Véronique, 75010 Paris (FR); Quentin, Eric, 93290 Tremblay en France (FR)
(74) Mandataire: Rasson, Catherine

(56) Documents cités:
- EP-A- 0 482 345
- EP-A- 0 803 245
- WO-A-97/17053
- US-A- 4 318 746
- DATABASE WPI Section Ch, Week 7438 Derwent Publications Ltd., London, GB; Class A96, AN 74-66866V XP002079283 & JP 49 012046 A (OGAWO AND CO LTD) , 2 février 1974
- PATENT ABSTRACTS OF JAPAN vol. 013, no. 069 (C-569), 16 février 1989 & JP 63 260956 A (LION CORP), 27 octobre 1988

## Description

La présente invention concerne une composition solide à application topique ainsi que son utilisation dans les domaines cosmétique et/ou dermatologique, notamment pour le soin et/ou le traitement de la peau, du cuir chevelu, des cheveux ou des muqueuses, pour le maquillage de la peau et/ou des fibres kératiniques telles que les cils et les cheveux, pour le coiffage et/ou la mise en forme des fibres kératiniques, et en particulier des cheveux.

On connaît dans l'industrie cosmétique des produits se présentant sous forme solide. Comme produits de ce type, on peut citer par exemple dans le domaine du maquillage, les bâtons ou "sticks" de rouge à lèvres, de fond de teint ou d'ombre à paupières ; dans le domaine du soin de la peau ou des lèvres, les crayons réparateurs des lèvres, les bâtons ou "sticks" dépigmentants, démaquillants ou hydratants ; dans le domaine de l'hygiène, les sticks déodorants, les sticks ou les pains moussants pour le rasage ou pour le lavage de la peau.

Les sticks formulés à base de cires présentent certains inconvénients : ils ont un caractère gras qui n'est pas apprécié par les utilisateurs et ils manquent de fraîcheur à l'application. En outre, il est difficile d'y introduire des actifs hydrophiles.

Par ailleurs, les sticks non gras tels que les sticks déodorants contiennent généralement une quantité relativement importante de sels d'acides gras qui peuvent avoir un caractère irritant pour des applications telles que le soin du visage. Par ailleurs, ces sticks laissent un film collant après application sur la peau.

En outre, des gels rigides aqueux sont décrits dans les documents WO-A-97/17055 et WO-A-97/17053. Toutefois, ces gels nécessitent l'emploi d'une concentration assez élevée de gélifiant ou font appel à une technique de préparation particulière, l'extrusion. En outre, les sticks décrits dans le document WO-A-97/17055 manquent de transparence et, du fait de la concentration élevée en gélifiant, manquent de fraîcheur et de douceur lors de l'application sur la peau, et ceux décrits dans le document WO-A-97/17053 doivent être hydratés au moment de l'emploi.

Par ailleurs, le document EP-A-803245 décrit des compositions solides aqueuses contenant des polysaccharides thermoréversibles, un humectant et une phase poudreuse (charges). Toutefois, la présence d'une phase poudreuse peut entraîner les inconvénients suivants : présence d'une trace visible après application de la composition sur la peau et diminution du confort. En outre, quand on enlève la phase poudreuse de la composition décrite dans le document EP-A-803245, on obtient une composition n'ayant ni une solidité ni une stabilité suffisantes, et ne donnant pas sur la peau un transfert satisfaisant.

Aussi, il subsiste le besoin d'une composition solide à application topique, ne présentant pas les inconvénients de l'art antérieur.

La demanderesse a découvert de façon inattendue un système gélifiant hydrophile particulier permettant de réaliser des compositions aqueuses rigides, homogènes et stables même à de faibles taux de gélifiant et même en l'absence de charges, et ne nécessitant pas obligatoirement l'intervention d'une technique de préparation particulière. Les compositions obtenues peuvent contenir une grande quantité d'eau et sont donc fraîches à l'application tout en donnant une impression de douceur. En outre, elles permettent l'application directe de produit sur la peau sans nécessiter de mouillage préalable et sans laisser de dépôt poudreux visible sur la peau.

La présente invention a pour objet une composition solide exempte de charges, contenant, dans une phase aqueuse, moins de 20 % du poids total de la composition, d'un système gélifiant hydrophile comprenant l'association d'au moins 2 % en poids de kappa-carraghénane, par rapport au poids total de la composition, et d'au moins un hydrocolloïde choisi parmi la gomme de xanthane et les dérivés cellulosiques solubles dans l'eau chaude.

On entend par "exempte de charges" le fait que la composition ne contient ni poudres ni pigments ni colorants insolubles. La composition de l'invention est notamment exempte de poudres telles que les poudres de talc, d'amidon, de polymères et copolymères d'acrylates, de mica, de kaolin, de polyamide (nylon), de polyéthylène, de silice et de silicone.

Par ailleurs, on entend par "hydrocolloïde" une macromolécule soluble dans l'eau, ne modifiant pas la valeur de l'activité en eau de la composition le contenant.

En outre, on entend par composition solide, toute composition présentant une résistance à la compression supérieure ou égale à 20 grammes, à température ambiante (20-25°C), après pénétration par une sonde cylindrique de révolution ayant un diamètre de 0,8 cm dans la matrice de la composition dans une épaisseur de 1 mm à une vitesse de 0,5 mm/s et retrait de ladite sonde de la matrice de la composition à une vitesse de 0,5 mm/s ; la résistance à la compression étant mesurée avec un analyseur du type "LFRA Texture Analyser" commercialisé par la Société STEVENS/MECHTRIC.

La composition solide obtenue est apte à s'appliquer directement sur un support, c'est-à-dire qu'elle n'a pas besoin d'être mouillée pour s'appliquer sur le support et notamment sur la peau. On entend par "support" de la composition selon l'invention, toute surface sur laquelle on peut faire une application topique, notamment la peau, les fibres kératiniques telles que les cils et les cheveux, le cuir chevelu et les muqueuses telles que les lèvres.

En outre, contrairement aux compositions solides comportant une forte proportion de gélifiants, la composition de l'invention a l'avantage de ne pas laisser de dépôt poudreux visible lorsqu'elle est appliquée sur un support. Par ailleurs, elle présente la propriété d'être transparente ou translucide en l'absence d'huile.

La kappa-carraghénane utilisée comme seul gélifiant à 2% donne une composition certes solide, mais friable, cassante et présentant de la synérèse (mauvaise stabilité dans le temps), donc peu adaptée à une application cosmétique ou dermatologique. En outre, en augmentant la quantité de kappa-carraghénane, on obtient une composition opaque qui s'applique mal sur un support tel que la peau, et présente de la synérèse. Seule l'association revendiquée permet d'obtenir une composition solide présentant de bonnes propriétés à la fois de solidité et de transfert.

Ainsi, la composition solide selon l'invention présente une bonne propriété de transfert, c'est-à-dire que lors d'une application sur un support, elle libère une quantité efficace de produit sur ce support.

La kappa-carraghénane utilisée dans la composition de l'invention peut être pure ou mélangée à une autre carraghénane telle que la iota-carraghénane.

La quantité de kappa-carraghénane est d'au moins 2 % du poids total de la composition, et elle peut aller par exemple de 2 à 15 %, de préférence de 2 à 8 % et mieux de 2 à 4 % du poids total de la composition.

Les dérivés cellulosiques utilisables dans la composition de l'invention doivent être solubles dans l'eau chaude. En effet, les dérivés cellulosiques insolubles dans l'eau chaude, tels que la méthylcellulose, l'hydroxypropylcellulose, l'hydroxypropylmethylcellulose et l'hydroxybutylcellulose, conduisent à des compositions hétérogènes qui, en outre, sont collantes et ne déposent pas de produit sur le support sur lequel elles sont appliquées. De telles compositions sont donc inaptes à résoudre le problème à la base de l'invention. La composition de l'invention est donc exempte de dérivé cellulosique insoluble dans l'eau chaude.

Comme dérivés cellulosiques solubles dans l'eau chaude, utilisables dans la composition de l'invention, on peut citer la carboxyméthylcellulose, l'hydroxyéthylcellulose, ainsi que les celluloses modifiées notamment par greffage de groupement alkyle. Selon un mode préféré de réalisation de l'invention, on utilise plus particulièrement la carboxyméthylcellulose.

Le ou les hydrocolloïdes associés à la kappa-carraghénane sont présents dans la composition selon l'invention, dans une quantité qui peut varier dans une large mesure. Ainsi, cette quantité peut aller par exemple de 1 à 10 %, de préférence de 1 à 5 % et mieux de 2 à 4 % du poids total de la composition.

Selon les proportions, et notamment lorsque la quantité totale du système gélifiant dépasse 4 %, on peut de manière avantageuse réaliser le mélange dans un extrudeur à deux vis selon la technique décrite dans le document EP-A-667148.

La phase aqueuse de la composition selon l'invention représente de 60 à 97 %, et de préférence de 80 à 95 % du poids total de la composition.

De façon avantageuse, la composition solide selon l'invention est une composition destinée à une application topique, notamment cosmétique ou dermatologique. Une telle composition comporte un milieu physiologiquement acceptable, en particulier pour la peau, les muqueuses, les ongles, les fibres kératiniques et/ou les cheveux.

Selon un mode particulier de l'invention, la composition comprend en outre au moins une huile, cette addition d'huile apportant un plus grand confort lors de l'application de la composition sur la peau.

Parmi les huiles utilisables, on peut citer les huiles minérales, les huiles d'origine végétale, les huiles d'origine animale, les huiles de synthèse telles que les esters gras, les huiles de silicone telles que les huiles de silicone volatile, les polyméthylsiloxanes, les polyméthylphénylsiloxanes, les polysiloxanes modifiés par des acides gras, des alcools gras ou des polyoxyalkylènes, les silicones fluorées et les huiles perfluorées. On peut ajouter d'autres corps gras tels que les acides gras, les alcools gras et les cires.

La ou les huiles et les autres corps gras éventuellement présents constituent la phase grasse.

La phase grasse peut être présente dans des proportions allant, par exemple, jusqu'à 30 %, de préférence de 0,1 à 20% et mieux de 0,5 à 10 % du poids total de la composition, ces proportions variant selon l'application choisie.

La phase grasse peut être introduite dans la phase aqueuse en présence de tensioactif pour assurer une meilleure dispersion.

Les compositions selon l'invention peuvent donc également contenir un ou plusieurs tensioactifs non ioniques, anioniques, cationiques ou amphotères, habituellement utilisés dans les domaines cosmétique et/ou dermatologique. Lorsqu'il est présent, la quantité d'agent tensioactif va de préférence de 0,05 à 8 % et mieux de 0,05 à 5 % du poids total de la composition.

Il est possible de modifier la rigidité des compositions selon l'invention en y ajoutant un ou plusieurs sels qui vont augmenter cette rigidité. Ces sels peuvent être choisis parmi les sels des métaux mono-, di- ou trivalents, et plus particulièrement les sels de métal alcalin et alcalino-terreux et en particulier les sels de sodium et de calcium. Les ions constituant ces sels peuvent être choisis par exemple parmi les carbonates, les bicarbonates, les sulfates, les glycérophosphates, les borates, les chlorures, les nitrates, les acétates, les hydroxydes, les persulfates ainsi que les sels d'α-hydroxyacides (citrates, tartrates, lactates, malates) ou d'acides de fruits, ou encore les sels d'acides aminés (aspartate, arginate, glycocholate, fumarate). La quantité de sel peut aller de 0,01 à 2 % et de préférence de 0,1 à 1 % du poids total de la composition.

De préférence, le sel est choisi parmi le nitrate de calcium, de magnésium ou de strontium, le borate de calcium ou de magnésium, le chlorure de calcium, de sodium, de magnésium, de strontium, de néodyme ou de manganèse, le sulfate de magnésium ou de calcium, l'acétate de calcium ou de magnésium, et leurs mélanges.

Les compositions selon l'invention peuvent contenir des additifs habituellement utilisés dans les domaines cosmétique et/ou dermatologique. On peut en particulier citer les agents antioxydants ou anti-radicaux libres, les colorants hydrosolubles tels que FD&C Red n°4 et D&C Green n°5, ou encore les colorants liposolubles si la composition comporte une phase grasse, les solvants, les actifs hydrophiles ou lipophiles, les parfums.

Les actifs peuvent être choisis par exemple parmi les agents hydratants ou humectants tels que les polyols et notamment la glycérine, les filtres UV, les agents antipelliculaires, les agents conditionneurs, les actifs déodorants, les dépigmentants ou les agents blanchissants, les agents tenseurs et antirides, les latex et les pseudolatex, et tout autre actif approprié à la finalité du produit solide considéré.

Comme solvants, on peut citer les alcools primaires tels que l'éthanol et l'isopropanol, les glycols tels que le propylène glycol, le butylène glycol, le dipropylène glycol, le diéthylène glycol, les éthers de glycol tel que les alkyl(C₁-C₄)éther de mono, di- ou tripropylène glycol, mono, di- ou triéthylène glycol, et leurs mélanges.

Comme latex et pseudolatex, on peut citer par exemple les dispersions de polymères synthétiques de type polycondensat ou de type radicalaire. Comme polymères constituant le latex ou le pseudolatex, on peut citer les polyuréthannes anioniques, cationiques, non ioniques ou amphotères, les polyuréthannes-acryliques, les polyuréthannes-polyvinylpyrrolidones, les polyester-polyuréthannes, les polyéther-polyuréthannes, les polyurées, les polymères acryliques, les copolymères acryliques, les polymères d'acide isophtalique sulfoné, ainsi que les polymères résultant de la polymérisation radicalaire d'un ou plusieurs monomères radicalaires. Comme polymère synthétique approprié pour être utilisé comme latex, on peut citer notamment les dispersions de polyester-polyuréthanne et de polyéther-polyuréthanne, commercialisées sous les dénominations "Sancure 2060" (polyester-polyuréthanne), "Sancure 2255" (polyester-polyuréthanne), "Sancure 815" (polyester-polyuréthanne), "Sancure 878" (polyéther-polyuréthanne) et "Sancure 861" (polyéther-polyuréthanne) par la société SANNCOR, sous les dénominations "Neorez R974" (polyester-polyuréthanne), "Neorez R981" (polyester-polyuréthanne), "Neorez R970" (polyéther-polyuréthanne) par la société ICI, et la dispersion de copolymère acrylique commercialisée sous la dénomination "Neocryl XK-90" par la société ZENECA.

Ces additifs peuvent être présents dans la composition finale en une quantité de 0 à 50 %, de préférence de 0,5 à 20 % et encore plus particulièrement entre 0,5 et 10 % du poids total de la composition.

Bien entendu, l'homme de l'art veillera à choisir ce ou ces éventuels additifs et/ou leurs quantités de manière telle que les propriétés avantageuses attachées intrinsèquement à la composition conforme à l'invention ne soient pas, ou substantiellement pas, altérées par la ou les adjonctions envisagées.

Les compositions selon l'invention peuvent constituer des produits pour le soin et/ou le conditionnement et/ou l'hygiène de la peau, des muqueuses, du cuir chevelu et/ou des cheveux. Parmi les produits de soin, de conditionnement ou d'hygiène en forme de pain, de stick ou de crayon, on peut mentionner, par exemple, en capillaire, des gels solides de coiffage et/ou de mise en forme des cheveux ; en soin de la peau, des produits hydratants, des produits amincissants, des produits dépigmentants et blanchissants, des produits pour le soin des lèvres ; pour l'hygiène du visage et/ou du corps, des produits de rasage, des déodorants.

Un autre objet de l'invention consiste en un procédé de traitement cosmétique pour le soin et/ou le conditionnement et/ou l'hygiène de la peau, des cheveux, du cuir chevelu et/ou des muqueuses, consistant à appliquer sur la peau, les cheveux, le cuir chevelu et/ou les muqueuses une composition solide telle que définie ci-dessus.

Les compositions selon l'invention peuvent constituer aussi des produits de maquillage, tels que des rouges à lèvres, des fonds de teints, des ombres à paupières, des fards à joues, des anti-cernes, des mascaras, des crayons du contour des lèvres, des crayons du contour des yeux, des sticks pour la teinture de mèches de cheveux. Elles peuvent notamment constituer des produits de maquillage "sans transfert", c'est-à-dire qui déposent un film qui ne transfère pas ou ne migre pas ou ne tache pas le support avec lequel le produit de maquillage appliqué sur la peau peut être mis en contact (vêtement, verre, tasse, etc...).

Aussi la présente invention a pour objet l'utilisation de la composition selon l'invention pour l'obtention d'un produit de maquillage sans transfert. Quand le produit de maquillage contient en outre un latex, on obtient un produit présentant une bonne rémanence.

La présente invention a encore pour objet un procédé de maquillage de la peau et/ou des fibres kératiniques, consistant à appliquer sur le visage, les lèvres, le contour des yeux, les joues, le contour des lèvres, les cils, les sourcils, les cheveux et/ou les paupières, une composition solide telle que définie ci-dessus.

Les exemples qui suivent servent à illustrer l'invention sans pour autant en limiter la portée. Les pourcentages sont exprimés en poids, sauf mention contraire.

### Exemple 1 : Stick hydratant

- Kappa-carraghénane 2 %
- Gomme de xanthane 2 %
- Eau qsp 100 %

Le stick est préparé par mélange des constituants à 80°C sous agitation et coulage à chaud.

### Exemple 2 : Rouge à lèvres

- Kappa-carraghénane 2 %
- Carboxyméthylcellulose 1 %
- FD&C Red n°4 0,3 %
- Eau qsp 100 %

Ce rouge à lèvres est préparé selon le même mode opératoire que dans l'exemple 1.

### Exemple 3 : Stick hydratant

- Kappa-carraghénane 2,75 %
- Carboxyméthylcellulose 2,75 %
- Glycérine 5 %
- Eau qsp 100 %

Ce stick est préparé selon le procédé décrit dans le document EP-A-667146 dans un extrudeur-cuiseur à deux vis (type "BC 21" de la société CLEXTRAL), dont la structure est la suivante :

| Entrée → → Sortie | | | | | | | | | | | |
|---|---|---|---|---|---|---|---|---|---|---|---|
| Structure de vis | DF | DF | BL | DF | BL | DF | BL | CF | DF | DF | DF |
| Longueur des manchons (mm) | 100 | 50 | 50 | 50 | 50 | 50 | 50 | 25 | 50 | 100 | 25 |
| Longueur des pas de vis (mm) | 33 | 25 | | 16,6 | | 16,6 | | -16,6 | 16,6 | 16,6 | 16,6 |

"DF" correspond à un filetage de vis hélicoïdale, dont le pas conduit la matière traitée de l'entrée vers la sortie du mélangeur ; "CF" correspond à un filetage de vis hélicoïdale à pas inverse du précédent (désigné avec une valeur de longueur de pas négative) qui repousse la matière traitée dans le sens allant de la sortie vers l'entrée du mélangeur, un tel filetage comportant des rainures longitudinales pour assurer le passage de la matière vers la sortie du mélangeur ; "BL" correspond à un tronçon bilobé comportant sur toute sa longueur une succession de lobes décalés de 90° les uns par rapport aux autres.

Conditions de l'extrusion :
- température de l'extrudeur : 100°C
- vitesse : 500 tours/minute
- débit : 3 kg/h

### Exemple 4 : Fond de teint

- Kappa-carraghénane 3 %
- Carboxyméthylcellulose 1 %
- FD&C Red n°4 0,2 %
- D&C Green n°5 0,1 %
- Huile végétale 5 %
- Eau qsp 100 %

Ce fond de teint est préparé selon le procédé de l'exemple 1.

## Revendications

1. Composition solide exempte de charges, contenant, dans une phase aqueuse, moins de 20 % du poids total de la composition, d'un système gélifiant hydrophile comprenant l'association d'au moins 2 % en poids de kappa-carraghénane par rapport au poids total de la composition, et d'au moins un hydrocolloïde choisi parmi la gomme de xanthane et les dérivés cellulosiques solubles dans l'eau chaude.

2. Composition selon la revendication 1, caractérisée en ce que le système gélifiant contient une autre carraghénane.

3. Composition selon la revendication 1 ou 2, caractérisée en ce que le dérivé cellulosique est choisi dans le groupe formé par la carboxyméthylcellulose, l'hydroxyéthylcellulose, les celluloses modifiées par greffage de groupement alkyle et leurs mélanges.

4. Composition selon l'une quelconque des revendications précédentes, caractérisée en ce que la kappa-carraghénane est présente en une quantité allant de 2 à 15 % et de préférence de 2 à 8 % du poids total de la composition.

5. Composition selon l'une quelconque des revendications précédentes, caractérisée en ce que l'hydrocolloïde associé à la kappa-carraghénane est présent en une quantité allant de 1 à 10 % et de préférence de 1 à 5 % du poids total de la composition.

6. Composition selon l'une quelconque des revendications précédentes, caractérisée en ce que la phase aqueuse représente de 60 à 97 % du poids total de la composition.

7. Composition selon l'une quelconque des revendications précédentes, caractérisée en ce qu'elle comprend en outre, au moins une phase grasse.

8. Composition selon la revendication précédente, caractérisée en ce que la phase grasse est présente en une quantité allant jusqu'à 30 % et de préférence de 0,1 à 20% du poids total de la composition.

9. Composition selon l'une quelconque des revendications précédentes, caractérisée en ce qu'elle contient en outre au moins un tensioactif.

10. Composition selon l'une quelconque des revendications précédentes, caractérisée en ce qu'elle contient en outre au moins un sel.

11. Composition selon l'une quelconque des revendications précédentes, caractérisée en ce qu'elle constitue une composition cosmétique ou dermatologique.

12. Composition selon l'une quelconque des revendications précédentes, caractérisée en ce qu'elle comprend, en outre, au moins un additif choisi dans le groupe formé par les agents antioxydants, les agents anti-radicaux libres, les colorants hydrosolubles ou liposolubles, les solvants, les actifs lipophiles ou hydrophiles, les parfums.

13. Composition selon la revendication précédente, caractérisée en ce que les actifs lipophiles ou hydrophiles sont choisis parmi les hydratants, les filtres UV, les agents antipelliculaires, les agents conditionneurs, les actifs déodorants, les dépigmentants, les agents tenseurs et antirides, les latex, les pseudolatex.

14. Produit de maquillage, caractérisé en ce qu'il comprend une composition selon l'une quelconque des revendications précédentes.

15. Produit pour le soin et/ou le conditionnement et/ou l'hygiène de la peau, des muqueuses, du cuir chevelu ou des cheveux, caractérisé en ce qu'il comprend une composition solide selon l'une quelconque des revendications 1 à 13.

16. Procédé de maquillage de la peau et/ou des fibres kératiniques, consistant à appliquer sur le visage, les lèvres, le contour des yeux, les joues, le contour des lèvres, les cils, les sourcils, les cheveux et/ou les paupières, une composition solide selon l'une quelconque des revendications 1 à 13.

17. Procédé de traitement cosmétique pour le soin et/ou le conditionnement et/ou l'hygiène de la peau, des cheveux, du cuir chevelu et/ou des muqueuses, consistant à appliquer sur la peau, les cheveux, le cuir chevelu et/ou les muqueuses une composition solide selon l'une quelconque des revendications 1 à 14.

18. Utilisation cosmétique de la composition selon l'une quelconque des revendications 1 à 13 pour obtenir un produit de maquillage sans transfert.

## Patentansprüche

1. Feste Zusammensetzung ohne Füllstoffe, die in einer wässerigen Phase bezogen auf das Gesamtgewicht der Zusammensetzung weniger als 20 % eines hydrophilen gelierenden Systems enthält, das mindestens 2 Gew.-% κ-Carrageenan, bezogen auf das Gesamtgewicht der Zusammensetzung, in Kombination mit mindestens einem Hydrokolloid enthält, das unter Xanthangummi und in warmem Wasser löslichen Cellulosederivaten ausgewählt ist.

2. Zusammensetzung nach Anspruch 1, dadurch gekennzeichnet, dass das gelierende System ein weiteres Carrageenan enthält.

3. Zusammensetzung nach Anspruch 1 oder 2, dadurch gekennzeichnet, dass das Cellulosederivat unter Carboxymethylcellulose, Hydroxyethylcellulose, durch Pfropfung von Alkylgruppen modifizierten Cellulosen und den Gemischen dieser Verbindungen ausgewählt ist.

4. Zusammensetzung nach einem der vorhergehenden Ansprüche, dadurch gekennzeichnet, dass das κ-Carrageenan in einer Menge im Bereich von 2 bis 15 % und vorzugsweise von 2 bis 8 % des Gesamtgewichts der Zusammensetzung vorliegt.

5. Zusammensetzung nach einem der vorhergehenden Ansprüche, dadurch gekennzeichnet, dass das Hydrokolloid, das mit dem κ-Carrageenan kombiniert ist, in einer Menge im Bereich von 1 bis 10 % und vorzugsweise von 1 bis 5 % des Gesamtgewichts der Zusammensetzung vorliegt.

6. Zusammensetzung nach einem der vorhergehenden Ansprüche, dadurch gekennzeichnet, dass die wässerige Phase 60 bis 97 % des Gesamtgewichts der Zusammensetzung ausmacht.

7. zusammensetzung nach einem der vorhergehenden Ansprüche, dadurch gekennzeichnet, dass sie ferner mindestens eine Fettphase enthält.

8. Zusammensetzung nach dem vorhergehenden Anspruch, dadurch gekennzeichnet, dass die Fettphase in einer Menge bis zu 30 % und vorzugsweise von 0,1 bis 20 % des Gesamtgewichts der Zusammensetzung vorliegt.

9. Zusammensetzung nach einem der vorhergehenden Ansprüche, dadurch gekennzeichnet, dass sie ferner mindestens einen grenzflächenaktiven Stoff enthält.

10. Zusammensetzung nach einem der vorhergehenden Ansprüche, dadurch gekennzeichnet, dass sie ferner mindestens ein Salz enthält.

11. Zusammensetzung nach einem der vorhergehenden Ansprüche, dadurch gekennzeichnet, dass es sich um eine kosmetische oder dermatologische Zusammensetzung handelt.

12. Zusammensetzung nach einem der vorhergehenden Ansprüche, dadurch gekennzeichnet, dass sie ferner mindestens einen Zusatzstoff enthält, der ausgewählt ist unter Antioxidantien, Mitteln gegen freie Radikale, wasserlöslichen oder fettlöslichen Färbemitteln, Lösemitteln, lipophilen oder hydrophilen Wirkstoffen und Parfums.

13. Zusammensetzung nach dem vorhergehenden Anspruch, dadurch gekennzeichnet, dass die lipophilen oder hydrophilen Wirkstoffe ausgewählt sind unter Hydratisierungsmitteln, UV-Filtern, Mitteln gegen Schuppen, Konditioniermitteln, desodorierenden Wirkstoffen, depigmentierenden Mitteln, straffenden Mitteln und Mitteln gegen Falten, Latices und Pseudolatices.

14. Schminkprodukt, dadurch gekennzeichnet, dass es eine Zusammensetzung nach einem der vorhergehenden Ansprüche enthält.

15. Produkt zur Pflege und/oder zum Konditionieren und/oder für die Hygiene der Haut, der Schleimhäute, der Kopfhaut oder der Haare, dadurch gekennzeichnet, dass es eine feste Zusammensetzung nach einem der Ansprüche 1 bis 13 enthält.

16. Verfahren zum Schminken der Haut und/oder der Keratinfasern, das darin besteht, eine feste Zusammensetzung nach einem der Ansprüche 1 bis 13 auf das Gesicht, die Lippen, die Augenkontur, die Wangen, die Lippenkontur, die Wimpern, die Augenbrauen, die Haare und/oder die Augenlider aufzubringen.

17. Verfahren zur kosmetisches Behandlung zur Pflege und/oder zum Konditionieren und/oder für die Hygiene der Haut, der Haare, der Kopfhaut und/oder der Schleimhäute, das darin besteht, eine feste Zusammensetzung nach einem der Ansprüche 1 bis 14 auf die Haut, die Haare, die Kopfhaut und/oder die Schleimhäute aufzubringen.

18. Kosmetische Verwendung der Zusammensetzung nach einem der Ansprüche 1 bis 13 zur Herstellung eines Schminkproduktes ohne Transfer.

## Claims

1. Solid composition free of fillers, containing, in an aqueous phase, less than 20% of the total weight of the composition of a hydrophilic gelling system comprising the combination of at least 2% by weight of kappa-carrageenan, relative to the total weight of the composition, and at least one hydrocolloid chosen from xanthan gum and cellulose derivatives which are soluble in hot water.

2. Composition according to Claim 1, characterized in that the gelling system contains another carrageenan.

3. Composition according to Claim 1 or 2, characterized in that the cellulose derivative is chosen from the group formed by carboxymethylcellulose, hydroxyethylcellulose and celluloses modified by grafting an alkyl group, and mixtures thereof.

4. Composition according to any one of the preceding claims, characterized in that the kappa-carrageenan is present in an amount ranging from 2 to 15%, and preferably from 2 to 8%, of the total weight of the composition.

5. Composition according to any one of the preceding claims, characterized in that the hydrocolloid combined with the kappa-carrageenan is present in an amount ranging from 1 to 10%, and preferably from 1 to 5%, of the total weight of the composition.

6. Composition according to any one of the preceding claims, characterized in that the aqueous phase represents from 60 to 97% of the total weight of the composition.

7. Composition according to any one of the preceding claims, characterized in that it also comprises at least one fatty phase.

8. Composition according to the preceding claim, characterized in that the fatty phase is present in an amount ranging up to 30%, and preferably from 0.1 to 20%, of the total weight of the composition.

9. Composition according to any one of the preceding claims, characterized in that it also contains at least one surfactant.

10. Composition according to any one of the preceding claims, characterized in that it also contains at least one salt.

11. Composition according to any one of the preceding claims, characterized in that it is a cosmetic or dermatological composition.

12. Composition according to any one of the preceding claims, characterized in that it also comprises at least one additive chosen from the group formed by antioxidants, anti-free-radical agents, water-soluble or liposoluble dyes, solvents, lipophilic or hydrophilic active agents and fragrances.

13. Composition according to the preceding claim, characterized in that the lipophilic or hydrophilic active agents are chosen from moisturizers, UV screening agents, antidandruff agents, conditioners, deodorants, depigmenting agents, tensioning agents, anti-wrinkle agents, latices and pseudolatices.

14. Make-up product, characterized in that it comprises a composition according to any one of the preceding claims.

15. Product for the care and/or conditioning and/or hygiene of the skin, mucous membranes, the scalp or the hair, characterized in that it comprises a solid composition according to any one of Claims 1 to 13.

16. Process for making up the skin and/or keratin fibres, which consists in applying a solid composition according to any one of Claims 1 to 13 to the face, the lips, the area around the eyes, the cheeks, the contour of the lips, the eyelashes, the eyebrows, the hair and/or the eyelids.

17. Cosmetic treatment process for the care and/or conditioning and/or hygiene of the skin, the hair, the scalp and/or mucous membranes, which consists in applying a solid composition according to any one of Claims 1 to 14 to the skin, the hair, the scalp and/or mucous membranes.

18. Cosmetic use of the composition according to any one of Claims 1 to 13 in order to obtain a transfer-resistant make-up product.
